# EUROPÄISCHE PATENTSCHRIFT

(11) **EP 3 570 788 B1**
(45) Veröffentlichungstag und Bekanntmachung des Hinweises auf die Patenterteilung: **10.03.2021**
(21) Anmeldenummer: 18701718.1
(22) Anmeldetag: 23.01.2018
(51) Int. Cl.: A61F 2/46, A61B 17/88, A61B 17/92

(54) **HÜFTGELENKENDOPROTHESENSYSTEM**
HIP JOINT ENDOPROSTHESIS SYSTEM
SYSTÈME D'ENDOPROTHÈSES DE HANCHES

(30) Priorität: 23.01.2017 DE 102017101191
(43) Veröffentlichungstag der Anmeldung: 27.11.2019
(73) Patentinhaber: Aesculap AG, 78532 Tuttlingen (DE)
(72) Erfinder: SUNGU, Mevlüt, 8200 Schaffhausen (CH); VARSANI, Anil, 78532 Tuttlingen/Möhringen (DE)
(74) Vertreter: Hoeger, Stellrecht & Partner Patentanwälte mbB
(86) Internationale Anmeldenummer: PCT/EP2018/051501
(87) Internationale Veröffentlichungsnummer: WO 2018/134423

(56) Entgegenhaltungen:
- EP-A1- 0 207 873
- EP-A2- 0 940 127
- WO-A1-90/13271
- WO-A1-2016/187305
- DE-A1- 4 439 309
- DE-U1- 9 212 846
- FR-A1- 2 770 128

## Beschreibung

Die vorliegende Erfindung betrifft ein Hüftgelenkendoprothesensystem umfassend ein medizinisches Instrument zum Extrahieren eines implantierten Prothesenschaftes einer Hüftgelenkendoprothese aus einem Femurknochen eines Patienten, welches medizinische Instrument einen Kopplungskörper mit einer Kopplungsausnehmung zum Aufnehmen eines vom Prothesenschaft abstehenden Kopplungskonus aufweist, wobei der Kopplungskörper einen Kopplungsgrundkörper und einen in einer Kopplungseinsatzaufnahme des Kopplungsgrundkörpers aufgenommenen, die Kopplungsausnehmung definierenden Kopplungseinsatz umfasst wobei das medizinische Instrument eine Verbindungseinrichtung umfasst zum kraft- und/oder formschlüssigen Verbinden des Kopplungseinsatzes und des Kopplungsgrundkörpers in einer Verbindungsstellung.

Zur Behandlung geschädigter Hüftgelenke werden diese seit vielen Jahren durch Hüftgelenkendoprothesen ersetzt. Hierbei wird ein Prothesenschaft in eine dafür vorbereitete Kavität des Femurknochens eingesetzt. Bekannt sind Prothesenschäfte, die einen abstehenden Kopplungskonus aufweisen, welcher beispielsweise mit einer Halsverlängerung oder einer Gelenkkugel koppelbar ist. Eine weitere Komponente der Hüftgelenkendoprothese ist die Gelenkpfanne, die im Beckenknochen des Patienten fixiert wird. Die Hüftgelenkpfanne kann optional einen Gelenkeinsatz aufnehmen, der mit der Gelenkkugel eine möglichst verschleißarme Gleitpaarung ausbildet.

Wird beispielsweise im Rahmen einer Operation zur Implantation der Hüftgelenkendoprothese ein zu großer oder zu kleiner Prothesenschaft in die vorbereitete Knochenkavität eingesetzt, muss diese wieder entfernt werden. Zu diesem Zweck werden medizinische Instrumente eingesetzt, die einen Kopplungskörper aufweisen, welcher mit dem Kopplungskonus des Prothesenschafts koppelbar ist. Ein Problem dabei ist insbesondere, dass der Kopplungskonus im Zusammenwirken mit dem Kopplungskörper des medizinischen Instruments beschädigt werden kann. Dies ist absolut unerwünscht, denn ein beschädigter Kopplungskonus ermöglicht keine sichere Verbindung mehr mit einem Halsstück oder einer Gelenkkugel. Die Verbindung zwischen dem Kopplungskonus und dem Halsstück oder der Gelenkkugel erfolgt dabei über Selbsthemmung. Hierfür sind optimal aufeinander abgestimmte Oberflächen erforderlich. Kratzer und Erhebungen am Kopplungskonus verhindern eine sichere selbsthemmende Verbindung.

Aus der WO 90/13271 A1 ist eine Vorrichtung zum Handhaben von Prothesen bekannt. Verfahren und Vorrichtungen zum Extrahieren medizinischer Implantate sind in der WO 2016/187305 A1 beschrieben. Die DE 92 12 846 U1 offenbart ein Extraktionsgerät zur Explantation von Hüftschaft-Endoprothesen. Eine Hüftprothesen-Greifvorrichtung ist aus der DE 44 39 309 A1 bekannt. In der EP 0 207 873 A1 ist ein Prothesenhalter beschrieben. Hüftprothesensystem ist in der FR 2 770 128 A1 offenbart. Ein Instrument zum Einsetzen von Prothesen ist aus der EP 0 940 127 A2 bekannt.

Es ist daher eine Aufgabe der vorliegenden Erfindung, ein Hüftgelenkendoprothesensystem der eingangs beschriebenen Art so zu verbessern, dass Prothesenschäfte einfach und sicher aus einem Femurknochen eines Patienten extrahiert werden können.

Diese Aufgabe wird bei einem Hüftgelenkendoprothesensystem der eingangs beschriebenen Art erfindungsgemäß dadurch gelöst, dass, dadurch gekennzeichnet, dass die Verbindungseinrichtung in Form einer Rast- oder Schnappverbindungseinrichtung ausgebildet ist.

Die erfindungsgemäß vorgeschlagene Weiterbildung eines medizinischen Instruments der eingangs beschriebenen Art ermöglicht es insbesondere, die Kopplungsausnehmung durch ein Material zu begrenzen, welches weicher ist als der Kopplungskonus des Prothesenschafts. So können auf einfache und sichere Weise Beschädigungen am Kopplungskonus verhindert werden. Die mindestens zweiteilige Ausbildung des Kopplungskörpers durch einen Kopplungsgrundkörper und einen Kopplungseinsatz ermöglicht es zudem, den Kopplungseinsatz und den Kopplungsgrundkörper optional aus unterschiedlichen Materialien auszubilden. So kann der Kopplungseinsatz insbesondere aus einem Material ausgebildet werden, mit dem die Gefahr einer Beschädigung des Kopplungskonus minimiert wird. Der Kopplungsgrundkörper dagegen kann hinreichend stabil ausgebildet werden, um für die Extraktion des Prothesenschafts aufzubringende Kräfte zu übertragen und in den Kopplungskonus einzuleiten. Gemäß der Erfindung ist vorgesehen, dass das medizinische Instrument eine Verbindungseinrichtung umfasst zum kraft- und/oder formschlüssigen Verbinden des Kopplungseinsatzes und des Kopplungsgrundkörpers in einer Verbindungsstellung. Mit der Verbindungseinrichtung kann insbesondere eine definierte Verbindung zwischen dem Kopplungseinsatz und dem Kopplungsgrundkörper sichergestellt werden. Auf einfache und kostengünstige Weise ausbilden lässt sich das Hüftgelenkendoprothesensystem, weil die Verbindungseinrichtung in Form einer Rast- oder Schnappverbindungseinrichtung ausgebildet ist.

Günstigerweise ist der Kopplungsgrundkörper aus einem härteren Material ausgebildet ist als der Kopplungseinsatz. So lassen sich Beschädigungen des Kopplungskonus des Prothesenschafts durch den Kopplungskörper minimieren, wenn der Kopplungskonus lediglich mit dem Kopplungseinsatz in Kontakt kommen und zusammenwirken kann. Beispielsweise kann der Kopplungskonus aus einem Implantatstahl oder insbesondere aus Titan oder einer Titanlegierung ausgebildet sein. Der Kopplungseinsatz ist dann vorzugsweise aus einem Material ausgebildet, welches weicher ist als das Material, aus dem der Kopplungskonus ausgebildet ist.

Um eine hinreichende Stabilität des medizinischen Instruments zu gewährleisten, ist es vorteilhaft, wenn der Kopplungsgrundkörper aus einem Metall ausgebildet ist. Insbesondere kann der Kopplungsgrundkörper aus einem Instrumentenstahl, beispielsweise einer Kobalt-Chrom-Legierung ausgebildet sein.

Einfach und kostengünstig ausbilden lässt sich das Hüftgelenkendoprothesensystem, wenn der Kopplungseinsatz einem Kunststoff ausgebildet ist. Insbesondere kann es sich bei dem Kunststoff um Polyetheretherketon (PEEK) und/oder Polypropylen (PP) und/oder Polyphenylensulfon (PPSU) handeln. Die genannten Kunststoffe lassen sich insbesondere einfach und sicher sterilisieren und sind hinreichend weich, um eine Beschädigung des Kopplungskonus zu verhindern, jedoch hinreichend formstabil, um für die Extraktion des Prothesenschafts aus dem Femurknochen erforderliche Kräfte auf den Prothesenschaft übertragen zu können.

Günstig ist es, wenn das medizinische Instrument mindestens ein erstes Kopplungselement umfasst zum Koppeln mit einem Instrumentengriff. Dies ermöglicht es insbesondere, den Kopplungskörper in Form eines Adapters, auch Extraktionsadapter genannt, auszubilden, welcher mit einem Instrumentengriff gekoppelt werden kann. So ist es insbesondere möglich, mehrere Kopplungskörper mit unterschiedlich großen und unterschiedlich geformten Kopplungsausnehmungen bereitzustellen, jedoch nur einen Instrumentengriff. Ein Operateur kann dann beispielsweise den passenden Kopplungskörper auswählen, mit dem Kopplungskonus des Prothesenschafts in Eingriff bringen und dann mit dem Instrumentengriff koppeln, um den Prothesenschaft zu extrahieren. So lassen sich modulare Hüftgelenkendoprothesensysteme ausbilden, die eine minimale Anzahl von Teilen benötigen, um eine Vielzahl unterschiedlicher Prothesenschäfte zu extrahieren.

Auf einfache Weise ausbilden lässt sich der Kopplungsgrundkörper, wenn das erste Kopplungselement in Form eines Kopplungsvorsprungs ausgebildet ist. Dieser kann beispielsweise von einer korrespondierenden Kopplungselementaufnahme am Instrumentengriff aufgenommen werden.

Vorzugsweise umfasst das medizinische Instrument einen Instrumentengriff. Der Instrumentengriff kann insbesondere einstückig mit dem Kopplungsgrundkörper ausgebildet sein. Er kann jedoch wie beschrieben auch temporär mit dem Kopplungsgrundkörper koppelbar sein. Beispielswiese kann der Instrumentengriff in Form eines Raspelgriffs ausgebildet sein. Insbesondere kann der Raspelgriff wie in der DE 10 2008 064518 A1 beschrieben ausgebildet sein. Der Instrumentengriff kann insbesondere mit einem Raspelkörper zur Ausbildung einer chirurgischen Raspel gekoppelt werden. Mit der Raspel kann die Kavität im Femurknochen präpariert werden vor dem Einsetzen des Prothesenschafts der Hüftgelenkendoprothese. Ist die Knochenkavität in gewünschter Weise vorbereitet, kann der Raspelgriff vom Raspelkörper getrennt werden. Mit diesem Raspelgriff kann dann beispielsweise der Kopplungsgrundkörper gekoppelt werden durch in Eingriff Bringen des Raspelgriffs mit dem ersten Kopplungselement. Durch diese modulare Ausgestaltung lässt sich die Zahl der für eine Implantation einer Hüftgelenkendoprothese erforderlichen Instrumente weiter minimieren.

Vorteilhaft ist es, wenn der Instrumentengriff einen Schlagkörper mit einer Schlagfläche aufweist und wenn der Schlagkörper an einem proximalen Ende des Instrumentengriffs angeordnet oder ausgebildet ist oder das proximale Ende des Instrumentengriffs bildet. Ist der Instrumentengriff mit dem Kopplungskörper gekoppelt, können durch Beaufschlagen der Schlagfläche des Schlagkörpers, beispielsweise mit einem Hammer, Kraftstöße vom Instrumentengriff über den Kopplungskörper auf den mit dem medizinischen Instrument gekoppelten Prothesenschaft übertragen werden, um diesen aus dem Femurschaft zu lösen oder eine Verbindung zwischen dem Prothesenschaft und dem Femur des Patienten zu lockern.

Auf einfache Weise lässt sich der Instrumentengriff mit dem Kopplungskörper verbinden, wenn der Instrumentengriff mindestens ein zweites Kopplungselement umfasst zum kraft- und/oder formschlüssigen in Eingriff Bringen mit dem mindestens einen ersten Kopplungselement in einer Koppelstellung. Insbesondere können die ersten und zweiten Kopplungselemente korrespondierend zueinander ausgebildet und für eine optimale Kopplung aufeinander abgestimmt sein.

Vorzugsweise ist das mindestens eine zweite Kopplungselement in Form einer Kopplungselementaufnahme zum Aufnehmen des mindestens einen ersten Kopplungselements ausgebildet. Eine Kopplungselementaufnahme vorzusehen ist insbesondere dann vorteilhaft, wenn das erste Kopplungselement in Form eines zur Kopplungselementaufnahme korrespondierenden Kopplungsvorsprungs ausgebildet ist.

Insbesondere für minimalinvasive chirurgische Eingriffe ist es vorteilhaft, wenn das mindestens eine zweite Kopplungselement an einem distalen Ende des Instrumentengriffs angeordnet oder ausgebildet ist oder das distale Ende des Instrumentengriffs bildet. So lässt sich der Instrumentengriff einfach und sicher mit dem Kopplungskörper in Eingriff bringen.

Vorzugsweise ist die Kopplungsausnehmung ausgebildet ist zum kraft- und/ oder formschlüssigen Aufnehmen des Kopplungskonus. So kann eine stabile Verbindung zwischen dem Kopplungskörper und dem Kopplungskonus des Prothesenschafts ausgebildet werden, wodurch eine Extraktion des Prothesenschafts aus dem Femurknochen erleichtert wird.

Gemäß einer weiteren bevorzugten Ausführungsform der Erfindung kann vorgesehen sein, dass die Kopplungsausnehmung eine seitliche Einführöffnung aufweist zum Einschieben des Kopplungskonus in die Kopplungsausnehmung in einer Einführrichtung, welche quer, insbesondere senkrecht, zu einer durch den Kopplungskonus definierten Längsachse verläuft. Insbesondere kann die Längsachse durch die Symmetrieachse des Kopplungskonus definiert werden. So kann das medizinische Instrument mit dem Kopplungskörper neben den Kopplungskonus geführt und dann in der Einführrichtung über den Kopplungskonus geschoben werden, um diesen in der Kopplungsausnehmung aufzunehmen.

Ferner ist es vorteilhaft, wenn die Kopplungsausnehmung eine untere Einführöffnung aufweist, welche in einer Richtung quer, insbesondere senkrecht, zur Einführrichtung geöffnet ist. Die untere Einführöffnung ermöglicht es insbesondere, dass der Prothesenschaft nicht vom Kopplungskonus getrennt werden muss, um den Prothesenschaft zu extrahieren. Die untere Einführöffnung kann insbesondere durch einen Verbindungsbereich am Prothesenschaft durchsetzt werden, wenn das medizinische Instrument mit dem Kopplungskonus des Prothesenschafts in Eingriff steht. Der Verbindungsbereich verbindet den Kopplungskonus mit dem eigentlichen Schaft des Prothesenschafts. Insbesondere können dieser Schaft, der Verbindungsbereich und der Kopplungskonus einstückig ausgebildet sein zur Ausbildung des Prothesenschafts.

Vorteilhaft ist es ferner, wenn der Kopplungsgrundkörper den Kopplungseinsatz derart umgibt, dass die seitliche Einführöffnung und die untere Einführöffnung frei zugänglich sind, wenn der Kopplungseinsatz in die Kopplungseinsatzaufnahme eingesetzt ist. So kann der Kopplungskonus in die Kopplungsausnehmung eingeführt werden, wobei er lediglich mit dem Kopplungseinsatz in Kontakt kommen kann, nicht jedoch mit dem Kopplungsgrundkörper. So kann eine Beschädigung des Kopplungskonus durch den Kopplungsgrundkörper vermieden werden.

Vorzugsweise definiert der Kopplungseinsatz die seitliche Einführöffnung und/ oder die untere Einführöffnung begrenzende Kanten oder Übergangsbereiche. So wird insbesondere sichergestellt, dass beim Einführen des Kopplungskonus in die Kopplungsausnehmung der Kopplungskonus lediglich mit dem Kopplungseinsatz in Kontakt treten kann, wodurch die Gefahr einer Beschädigung des Kopplungskonus minimiert wird.

Um eine besonders gute Kraftübertragung zwischen dem medizinischen Instrument und dem Prothesenschaft zu erreichen, ist es vorteilhaft, wenn die Kopplungsausnehmung eine Innenkontur aufweist, welche mindestens abschnittsweise die Form eines Innenkonus aufweist. Insbesondere kann dieser Innenkonus korrespondierend zum Kopplungskonus ausgebildet sein, um mindestens teilweise eine flächige Anlage des Kopplungskonus an die Kopplungsausnehmung zu erreichen.

Auf einfache Weise koppeln lässt sich der Kopplungskörper mit dem Kopplungskonus, wenn der der Innenkonus eine Konuslängsachse definiert, welche die untere Einführöffnung durchsetzt.

Insbesondere können sich vom Prothesenschaft weg verjüngende Kopplungskonen mit dem Kopplungskörper in Eingriff gebracht werden, wenn ein Querschnitt des Innenkonus in Richtung auf die untere Einführöffnung hin zunimmt.

Um insbesondere Zug- oder Druckkräfte auf den Prothesenschaft mit dem medizinischen Instrument ausüben zu können, ist es vorteilhaft, wenn zwischen dem Innenkonus und der unteren Einführöffnung eine Querschnittsverengung ausgebildet ist. Die Querschnittsverengung kann insbesondere in den Übergangsbereich zwischen dem Kopplungskonus und dem Schaft des Prothesenschafts eingreifen, welcher in Form einer Einschnürung ausgebildet sein kann. Dadurch kann insbesondere die Querschnittsverengung am Kopplungskörper seitlich in die Einschnürung am Prothesenschaft eingeführt werden. Die Querschnittsverengung kann beispielsweise in Form eines in Richtung auf die Konuslängsachse hin weisenden Ringvorsprungs ausgebildet sein, welcher nicht vollständig in sich geschlossen ist.

Vorteilhaft ist es, wenn die Kopplungseinsatzaufnahme eine seitliche Kopplungseinsatzaufnahmeöffnung und eine untere Kopplungseinsatzaufnahmeöffnung aufweist. So kann der Kopplungseinsatz durch die seitliche Kopplungseinsatzaufnahmeöffnung in die Kopplungseinsatzaufnahme eingeführt werden. Die untere Kopplungseinsatzaufnahmeöffnung ermöglicht es insbesondere, dass die untere Einführöffnung des Kopplungseinsatzes frei zugänglich bleibt.

Vorzugsweise ist die seitliche Kopplungseinsatzaufnahmeöffnung ausgebildet zum Einschieben des Kopplungseinsatzes in die Kopplungseinsatzaufnahme.

So lassen sich der Kopplungseinsatz und der Kopplungsgrundkörper einfach und sicher miteinander in Eingriff bringen.

Um insbesondere Zugkräfte sicher mit dem medizinischen Instrument auf den Prothesenschaft übertragen zu können, ist es günstig, wenn die Kopplungseinsatzaufnahme eine Hinterschneidung aufweist, welche durch eine Verringerung eines freien Querschnitts der Kopplungseinsatzaufnahme in Richtung auf die untere Kopplungseinsatzaufnahmeöffnung hin gebildet ist. So können insbesondere Kräfte in axialer Richtung, also insbesondere parallel zur Konuslängsachse, vom Kopplungsgrundkörper auf den Kopplungseinsatz übertragen werden, ohne dass sich der Kopplungseinsatz aus dem Kopplungsgrundkörper lösen kann.

Günstigerweise ist der Kopplungseinsatz in der Kopplungseinsatzaufnahme kraft- und/oder formschlüssig gehalten. Dies ermöglicht es insbesondere, den Kopplungseinsatz vom Kopplungsgrundkörper zu trennen, beispielsweise zum Reinigen des medizinischen Instruments.

Auf einfache Weise ausbilden lässt sich die Verbindungseinrichtung, wenn sie erste und zweite Verbindungselemente umfasst, welche einerseits am Kopplungseinsatz und andererseits am Kopplungsgrundkörper ausgebildet sind, und wenn die ersten und zweiten Verbindungselemente in der Verbindungsstellung kraft- und/oder formschlüssig in Eingriff stehen.

Vorteilhaft ist es, wenn das erste Verbindungselement in Form einer Verbindungselementausnehmung ausgebildet ist und wenn das zweite Verbindungselement in Form eines Verbindungsvorsprungs ausgebildet ist. Insbesondere sind die ersten und zweiten Verbindungselemente korrespondierend zueinander ausgebildet. Sie können zudem wahlweise am Kopplungsgrundkörper und am Kopplungseinsatz ausgebildet sein.

Günstig ist es, wenn sich das erste und/oder das zweite Verbindungselement parallel oder im Wesentlichen parallel zur seitlichen Kopplungseinsatzaufnahmeöffnung und/oder zur seitlichen Einführöffnung erstrecken. Dies kann insbesondere so zu verstehen sein, dass die seitliche Einführöffnung eine Einführöffnungsebene definiert und sich die Verbindungselemente parallel zu dieser erstrecken. Dies hat insbesondere den Vorteil, dass durch die miteinander in Eingriff stehenden Verbindungselemente der Kopplungseinsatz nicht ohne zusätzliche Extraktionskräfte aus der Kopplungseinsatzaufnahme heraus bewegt werden kann. Insbesondere können sich also die Verbindungselemente quer zur Einführrichtung erstrecken und eine Relativbewegung zwischen dem Kopplungseinsatz und dem Kopplungsgrundkörper verhindern.

Auf einfache Weise ausbilden lässt sich das medizinische Instrument, wenn das am Kopplungsgrundkörper in der Kopplungseinsatzaufnahme ausgebildete Verbindungselement in Richtung auf die untere Kopplungseinsatzaufnahmeöffnung weisend angeordnet oder ausgebildet ist. Beispielsweise kann dieses Verbindungselement als Verbindungsvorsprung oder als Verbindungselementaufnahme ausgebildet sein, zum Beispiel als Rippe oder Nut.

Um das medizinische Instrument insbesondere bei minimalinvasiven chirurgischen Eingriffen einsetzen zu können, ist es vorteilhaft, wenn das erste Kopplungselement am Kopplungsgrundkörper derart angeordnet ist, dass es entgegen oder im Wesentlichen entgegen der unteren Kopplungseinsatzaufnahmeöffnung weist. Beispielsweise kann das erste Kopplungselement in Form eines Kopplungsvorsprungs ausgebildet sein, der vom Kopplungsgrundkörper absteht und in eine Richtung entgegen der Richtung weist, in die die untere Kopplungseinsatzaufnahmeöffnung hin weisend geöffnet ist.

Gemäß einer weiteren bevorzugten Ausführungsform der Erfindung kann vorgesehen sein, dass das Hüftgelenkendoprothesensystem ein Löseinstrument zum Lösen des Kopplungseinsatzes aus der Kopplungseinsatzaufnahme des Kopplungsgrundkörpers umfasst. Das Löseelement ermöglicht es insbesondere, den Kopplungseinsatz und den Kopplungsgrundkörper voneinander zu trennen, um diese zu reinigen und zu sterilisieren.

Günstig ist es, wenn das Löseinstrument ein Löseglied umfasst, wenn der Kopplungsgrundkörper eine Lösegliedöffnung umfasst und wenn das Löseglied eine Länge aufweist, welche größer ist als eine Dicke des Kopplungsgrundkörpers im Bereich der Lösegliedöffnung. Diese Ausgestaltung ermöglicht es insbesondere, dass durch Einführen des Löseglieds in die Lösegliedöffnung das Löseglied derart in die Kopplungseinsatzaufnahme hineinragen kann, dass eine Druckkraft auf den Kopplungseinsatz ausgeübt werden kann, vorzugsweise in einer Richtung parallel zur Einführrichtung, wodurch der Kopplungseinsatz aus der Kopplungseinsatzaufnahme herausgeschoben beziehungsweise herausgedrückt werden kann. Insbesondere können so die zum Lösen der Verbindungseinrichtung erforderlichen Kräfte, also zum außer Eingriff Bringen der miteinander in Eingriff stehenden ersten und zweiten Verbindungselemente, auf den Kopplungseinsatz übertragen werden.

Auf besonders einfache Weise lässt sich der Kopplungseinsatz aus dem Kopplungsgrundkörper lösen, wenn die Lösegliedöffnung der seitlichen Kopplungseinsatzaufnahmeöffnung gegenüberliegend angeordnet oder ausgebildet ist. So lässt sich der Kopplungseinsatz direkt aus der Kopplungseinsatzaufnahme durch die seitliche Kopplungseinsatzaufnahmeöffnung hindurch herausdrücken.

Auf einfache Weise ausbilden lässt sich das Löseelement, wenn das Löseglied in Form eines vom Löseinstrument abstehenden Lösezapfens ausgebildet ist.

Die zwischen den in Eingriff stehenden Verbindungselementen der Verbindungseinrichtung wirkenden Kräfte können besonders einfach überwunden werden zum Lösen des Kopplungseinsatzes und des Kopplungsgrundkörpers voneinander, wenn das Löseglied mit einem Außengewinde versehen ist und wenn die Lösegliedöffnung mit einem zum Außengewinde korrespondierenden Innengewinde versehen ist. So kann das Löseglied in die Lösegliedöffnung eingeschraubt werden, wodurch eine Lösekraft sehr dosiert vom Löseinstrument auf den Kopplungseinsatz übertragen werden kann.

Gemäß einer weiteren bevorzugten Ausführungsform der Erfindung kann vorgesehen sein, dass das Hüftgelenkendoprothesensystem einen einen abstehenden Kopplungskonus aufweisenden Prothesenschaft einer Hüftgelenkendoprothese umfasst. Insbesondere kann das Hüftgelenkendoprothesensystem mehrere unterschiedliche derartige Prothesenschäfte umfassen. Das Hüftgelenkendoprothesensystem kann passend zu jedem Kopplungskonus der Prothesenschäfte korrespondierende Kopplungskörper umfassen, so dass ein modulares Hüftgelenkendoprothesensystem ausgebildet werden kann.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische Gesamtansicht eines erfindungsgemäßen Hüftgelen kendoprothesensystems ;
- Figur 2:: eine teilweise geschnittene Ansicht der Anordnung aus Figur 1 längs Linie 2-2;
- Figur 3:: eine Seitenansicht eines mit einem Prothesenschaft gekoppelten Koppl u ngskörpers;

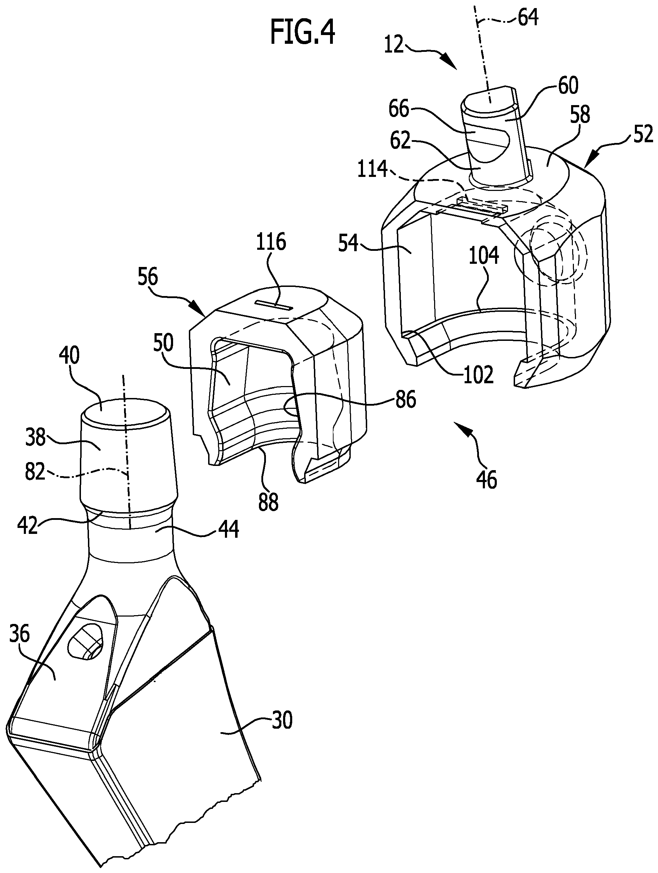

- Figur 4:: eine Explosionsdarstellung des Prothesenschafts, des Kopplungsgrundkörpers und des Kopplungseinsatzes;

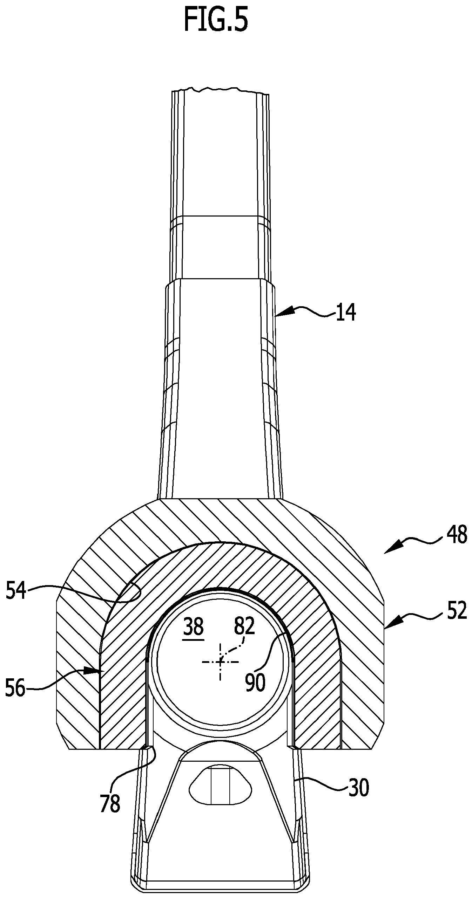

- Figur 5:: eine Schnittansicht längs Linie 5-5 in Figur 3; und

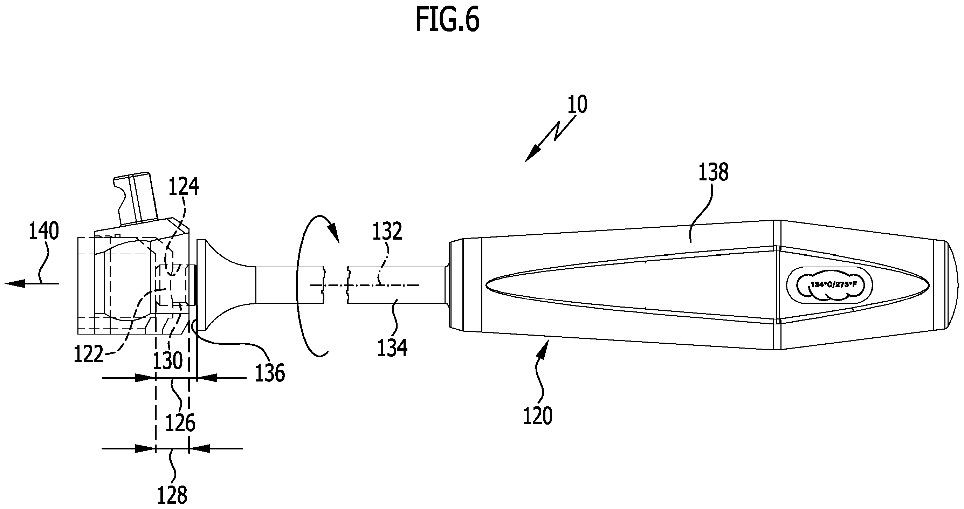

- Figur 6:: eine teilweise durchbrochene Seitenansicht des Kopplungskörpers beim Herauslösen des Kopplungseinsatzes aus dem Kopplungsgrundkörper mit einem Löseelement.

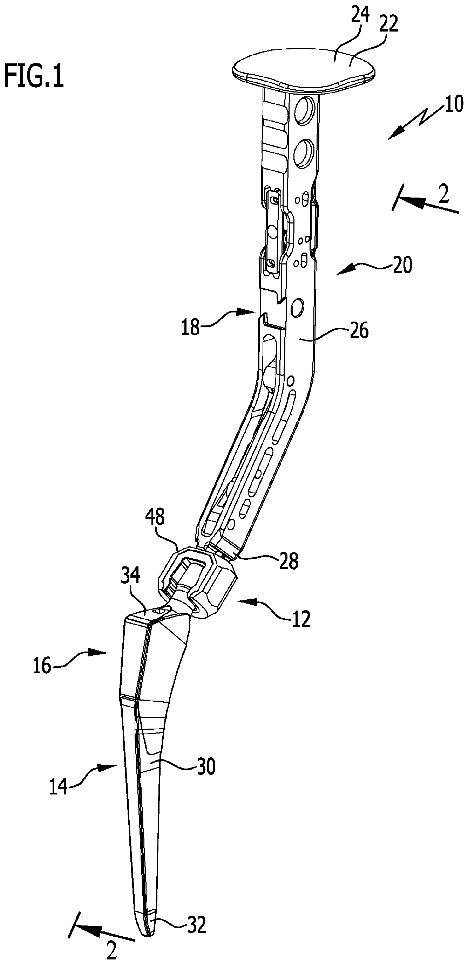

In Figur 1 ist ein erstes Ausführungsbeispiel eines insgesamt mit dem Bezugszeichen 10 bezeichneten Hüftgelenkendoprothesensystems schematisch dargestellt. Es umfasst ein medizinisches Instrument 12 zum Extrahieren eines implantierten Prothesenschafts 14 einer Hüftgelenkendoprothese 16 aus einem Femurknochen eines Patienten.

Optional umfasst das Hüftgelenkendoprothesensystem 10 ferner den Prothesenschaft 14 und weiter optional einen Instrumentengriff 18. Dieser kann insbesondere in Form eines Raspelgriffs 20 ausgebildet sein, wie er beispielsweise in der DE 10 2008 064 518 A1 beschrieben ist.

Der Instrumentengriff 18 weist an seinem proximalen Ende einen Schlagkörper 22 mit einer in proximaler Richtung weisenden, schwach vom Instrumentengriff 18 weg weisend konvex gekrümmten Schlagfläche 24 auf. Er bildet somit, wie in Figur 1 beispielhaft dargestellt, das proximale Ende des Instrumentengriffs 18.

Der Instrumentengriff 18 weist einen länglichen, abgeknickten Instrumentenkörper 26 auf, welcher sich vom Schlagkörper 22 zu einem distalen Ende des Instrumentengriffs 28 erstreckt.

Der Prothesenschaft 14 umfasst einen langgestreckten Schaftkörper 30, welcher sich zu einem distalen Ende 32 hin im Querschnitt verjüngt.

Von einem proximalen Ende 34 des Schaftkörpers 30 steht bezüglich einer Endfläche 36 desselben geneigt ein Kopplungskonus 38 ab. Der Kopplungskonus 38 ist in Form eines Kegelstumpfes ausgebildet, der sich in Richtung auf eine kreisförmige, vom Schaftkörper 30 weg weisende Endfläche 40 hin im Querschnitt verjüngt.

An ein distales Ende 42 des Kopplungskonus 38 schließt sich ein kurzer Halsabschnitt 44 an, welcher im Querschnitt am distalen Ende 42 des Kopplungskonus 38 verringert ist und so zwischen dem Kopplungskonus 38 und dem Schaftkörper 30 eine Einschnürung bildet.

Der Kopplungskonus 38 dient bei der Hüftgelenkendoprothese 16 zum Verbinden mit einer in den Figuren nicht dargestellten Gelenkkugel, die einen zum Kopplungskonus 38 korrespondierenden Aufnahmekonus aufweist, so dass der Prothesenschaft 14 und Gelenkkugel durch Selbsthemmung miteinander verbunden werden können. Optional kann, falls erforderlich der Kopplungskonus 38 auch mit einem nicht dargestellten Verlängerungsabschnitt in der beschriebenen Weise wie mit der Gelenkkugel gekoppelt werden, wobei der Verlängerungsabschnitt wiederum mit der Gelenkkugel gekoppelt werden kann.

Ist der Prothesenschaft 14 in eine dafür vorbereitete Kavität des Femurknochens des Patienten eingesetzt, passt jedoch nicht wie vom Operateur gewünscht, muss dieser den Prothesenschaft 14 wieder aus dem Femurknochen extrahieren. Hierzu dient das Instrument 12. Dieses ist in Form eines zweiteiligen Extraktionsadapters 46 ausgebildet und dient als Kopplungskörper 48 mit einer Kopplungsausnehmung 50 zum Aufnehmen des Kopplungskonus.

Der Kopplungskörper 48 umfasst einen Kopplungsgrundkörper 52 mit einer Kopplungseinsatzaufnahme 54, in welcher ein Kopplungseinsatz 56 kraft- und/oder formschlüssig aufgenommen ist. Die Kopplungsausnehmung 50 ist am Kopplungseinsatz 56 ausgebildet.

Der Kopplungsgrundkörper 52 ist aus einem härteren Material ausgebildet als der Kopplungseinsatz 56. Beispielsweise kann der Kopplungsgrundkörper 52 aus einem Metall ausgebildet sein, insbesondere aus einem Instrumentenstahl.

Der Kopplungseinsatz 56 ist vorzugsweise aus einem Material ausgebildet, das weicher ist als das Material, aus dem der Prothesenschaft 14 ausgebildet ist, insbesondere dessen Kopplungskonus 38. So kann das Risiko minimiert werden, dass der Kopplungskonus 38 beim Aufnehmen in die Kopplungsausnehmung 50 des Extraktionsadapters 46 beschädigt werden kann. Vorzugsweise ist der Kopplungseinsatz 56 aus einem Kunststoff ausgebildet. Beispielsweise kann dieser Kunststoff Polyetheretherketon (PEEK), Polypropylen (PP) und/oder Polyphenylensulfon (PPSU) sein. PEEK weist hier insbesondere die am besten geeigneten mechanischen Werkstoffrichtwerte auf, insbesondere Zug- und Druckfestigkeit, Härte und Elastizitätsmodul.

Um das Instrument 12 mit dem Instrumentengriff 18 koppeln zu können, ist am Kopplungskörper 48 an einer in proximaler Richtung weisenden Endfläche 58 ein erstes Kopplungselement 60 angeordnet oder ausgebildet. Dieses kann insbesondere, wie schematisch in den Figuren dargestellt, in Form eines Kopplungsvorsprungs 62 ausgebildet sein, welcher eine Kopplungsvorsprunglängsachse 64 definiert, die quer zur Endfläche 58 verläuft, insbesondere senkrecht zu dieser. Am Kopplungsvorsprung 62 ist seitlich eine Ausnehmung 66 ausgebildet, die eine Hinterschneidung definiert.

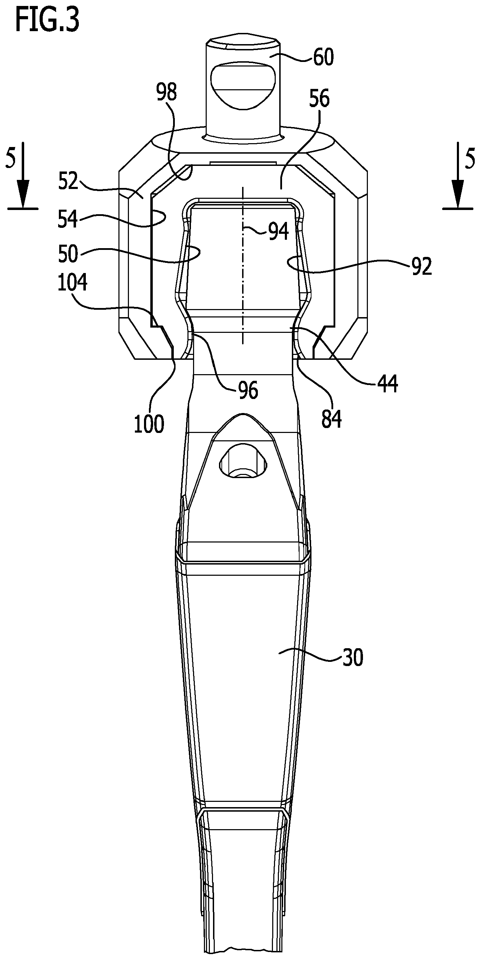

Am Instrumentengriff 18 ist ein zum ersten Kopplungselement 60 korrespondierendes zweites Kopplungselement 68 ausgebildet zum kraft- und/oder formschlüssigen in Eingriff Bringen mit dem ersten Kopplungselement 60 in einer Koppelstellung, die beispielhaft in den Figuren 1 bis 3 dargestellt ist. Das zweite Kopplungselement 68 ist in Form einer Kopplungselementaufnahme 70 zum Aufnehmen des ersten Kopplungselements 60 ausgebildet. Die Kopplungselementaufnahme 70 ist korrespondierend zum Kopplungsvorsprung 62 ausgebildet und nimmt diesen formschlüssig oder im Wesentlichen formschlüssig auf.

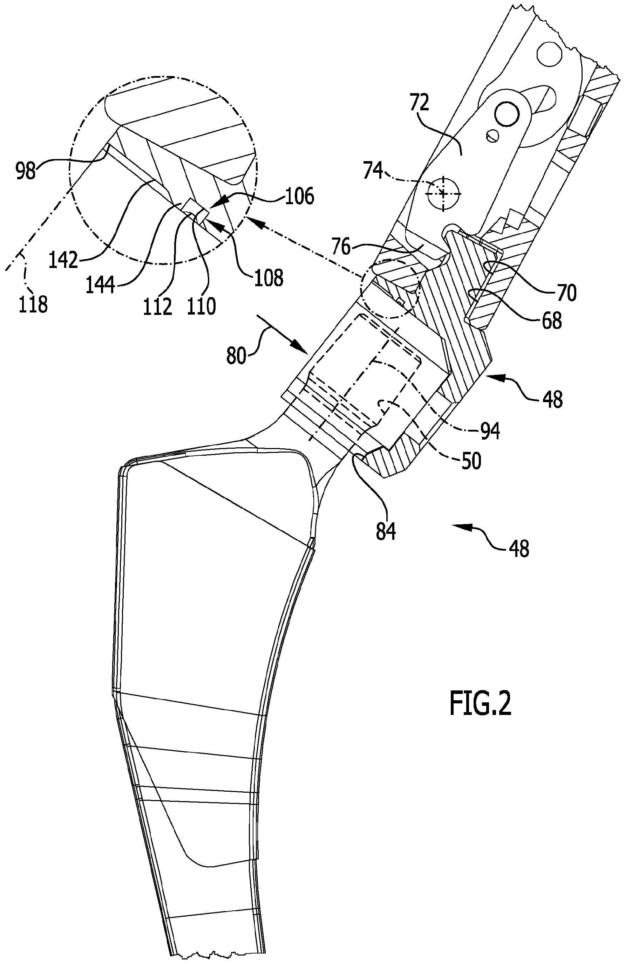

Zum Sichern des Extraktionsadapters 46 am Instrumentengriff 18 dient ein schwenkbares Sicherungsglied 72, welches am Instrumentengriff 18 um eine quer zur Kopplungsvorsprunglängsachse 64 verlaufende Schwenkachse 74 verschwenkbar gelagert ist. Ein distalseitiges Ende des Sicherungsglieds 72 ist in Form einer Verriegelungsnase 76 ausgebildet, die in einer Verriegelungsstellung in die Ausnehmung 66 eingeschwenkt ist, wie beispielhaft in Figur 2 dargestellt, und in einer Lösestellung, in welcher der Instrumentengriff 18 vom Extraktionsadapter 46 abgenommen werden kann, ausgeschwenkt ist.

Das Sicherungsglied 72 ist mit einem entsprechenden Schwenkmechanismus am Instrumentengriff 18 gekoppelt, um es um die Schwenkachse 74 zum Verbinden mit oder zum Lösen von dem ersten Kopplungselement 60 zu verschwenken. Der Schwenkmechanismus kann insbesondere wie in der DE 10 2008 064 518 A1 beschrieben ausgebildet sein.

Das zweite Kopplungselement 68 ist am distalen Ende 28 des Instrumentengriffs 18 angeordnet beziehungsweise bildet das Ende 28.

Die Kopplungsausnehmung 50 ist ausgebildet zum kraft- und/oder formschlüssigen Aufnehmen des Kopplungskonus 38. Sie weist eine seitliche Einführöffnung 78 auf zum Einschieben des Kopplungskonus 38 in die Kopplungsausnehmung 50 in einer Einführrichtung, die in Figur 2 schematisch durch den Pfeil 80 angegeben ist. Die Einführrichtung 80 verläuft quer, insbesondere senkrecht, zu einer durch den Kopplungskonus 38 definierten Längsachse 82.

Die Kopplungsausnehmung 50 weist ferner eine untere Einführöffnung 84 auf, welche in einer Richtung quer, insbesondere senkrecht, zur Einführrichtung 80 geöffnet ist.

Der Kopplungsgrundkörper 52 ist schalenartig oder in Form einer Schale ausgebildet und umgibt den Kopplungseinsatz 56 derart, dass die seitliche Einführöffnung 78 und die untere Einführöffnung 84 frei zugänglich sind, wenn der Kopplungseinsatz 56 in der Kopplungseinsatzaufnahme 54 aufgenommen ist. Diese Ausgestaltung ist insbesondere in den Figuren 1 bis 3 gut zu erkennen.

Der Kopplungseinsatz 56 definiert die seitliche Einführöffnung 78 sowie die untere Einführöffnung 84 begrenzende Kanten 86 beziehungsweise 88.

Die Kopplungsausnehmung 50 weist eine Innenkontur 90 auf, welche mindestens abschnittsweise die Form eines Innenkonus 92 aufweist.

Der Innenkonus 92 definiert eine Konuslängsachse 94, die die untere Einführöffnung 84 durchsetzt. Der Innenkonus 92 weist ferner einen Querschnitt auf, der in Richtung auf die untere Einführöffnung 84 hin zunimmt.

Die Kopplungsausnehmung 50 ist ferner derart ausgebildet, dass zwischen dem Innenkonus 92 und der unteren Einführöffnung 84 eine Querschnittsverengung 96 ausgebildet ist. Ist der Prothesenschaft 14 mit dem Extraktionsadapter 46 gekoppelt, greift die Querschnittsverengung 96, die quasi einen in Richtung auf die Konuslängsachse 94 hin konvex gekrümmten Ringvorsprung bildet, in den Halsabschnitt 44 zwischen dem Kopplungskonus 38 und dem Schaftkörper 30 ein.

Die Kopplungseinsatzaufnahme 54 weist eine seitliche Kopplungseinsatzaufnahmeöffnung 98 und eine untere Kopplungseinsatzaufnahmeöffnung 100 auf. Der Kopplungseinsatz 56 kann durch die entsprechend ausgebildete seitliche Kopplungseinsatzaufnahmeöffnung 98 in die Kopplungseinsatzaufnahme 54 ebenfalls in der Einführrichtung 80 eingeschoben werden.

Zur Sicherung des Kopplungseinsatzes 56 in der Kopplungseinsatzaufnahme in axialer Richtung, also parallel zur Konuslängsachse 94, dient eine Hinterschneidung 102, die durch eine in proximale Richtung weisende, in sich nicht geschlossene Ringfläche 104 begrenzt ist. Die Hinterschneidung 102 ist gebildet durch eine Verringerung eines freien Querschnitts der Kopplungseinsatzaufnahme 54 in Richtung auf die untere Kopplungseinsatzaufnahmeöffnung 100 hin.

Eine Außenkontur des Kopplungseinsatzes 56 ist an eine Innenkontur der Kopplungseinsatzaufnahme 54 im Wesentlichen angepasst, so dass der Kopplungseinsatz 56 in der Kopplungseinsatzaufnahme 54 kraft- und/oder formschlüssig gehalten ist.

Zum Sichern des Kopplungseinsatzes 56 in der Kopplungseinsatzaufnahme 54 gegen eine Bewegung entgegen der Einführrichtung 80 ist am medizinischen Instrument 12 eine Verbindungseinrichtung 106 vorgesehen, so dass der Kopplungseinsatz 56 und der Kopplungsgrundkörper 52 in einer in Figur 2 beispielhaft dargestellten Verbindungsstellung und kraft- und/oder formschlüssig miteinander verbunden sind.

Die Verbindungseinrichtung 106 kann insbesondere in Form einer Rast- oder Schnappverbindungseinrichtung 108 ausgebildet sein.

Die Verbindungseinrichtung 106 umfasst ein erstes Verbindungselement 110 und ein zweites Verbindungselement 112, die einerseits am Kopplungsgrundkörper 52 und andererseits am Kopplungseinsatz 56 angeordnet oder ausgebildet sind. In der Verbindungsstellung stehen die Verbindungselemente 110 und 112 kraft- und/oder formschlüssig in Eingriff.

Das erste Verbindungselement 110 ist in Form einer Verbindungselementausnehmung 114 ausgebildet. Das zweite Verbindungselement 112 ist in Form eines Verbindungsvorsprungs 116 ausgebildet.

Die Verbindungselemente 110 und 112 erstrecken sich parallel zu einer von der seitlichen Kopplungseinsatzaufnahmeöffnung 98 definierten Öffnungsebene 118, die ebenfalls von der seitlichen Einführöffnung 78 definiert ist.

Die langgestreckt quaderförmig ausgebildete Verbindungselementausnehmung 114 ist in Richtung auf die untere Kopplungseinsatzaufnahmeöffnung 100 hin weisend angeordnet beziehungsweise ausgebildet.

Ferner ist die untere Kopplungseinsatzaufnahmeöffnung 100 in einer Richtung weisend geöffnet, die der vom ersten Kopplungselement 60 definierten Richtung entgegengerichtet ist.

Das Hüftgelenkendoprothesensystem 10 kann ferner ein Löseinstrument 120 zum Lösen des Kopplungseinsatzes 56 aus der Kopplungseinsatzaufnahme 54 des Kopplungsgrundkörpers 52 umfassen. Es umfasst ein Löseglied 122.

Am Kopplungsgrundkörper 52 ist eine Lösegliedöffnung 124 ausgebildet. Eine Länge 126 des Löseglieds 122 ist größer als eine Dicke 128 des Kopplungsgrundkörpers 52 im Bereich der Lösegliedöffnung 124. Die Lösegliedöffnung 124 ist der seitlichen Kopplungseinsatzaufnahmeöffnung 98 gegenüberliegend angeordnet beziehungsweise ausgebildet.

Das Löseglied 122 ist in Form eines Lösezapfens 130 ausgebildet, der von einer quer zu einer Schaftlängsachse 132 eines Schafts 134 des Löseinstruments 120 verlaufenden Endfläche 136 senkrecht absteht.

Ein proximales Ende des Löseinstruments 120 bildet einen Griff 138.

Das Löseglied 122 ist vorzugsweise mit einem Außengewinde versehen, die Lösegliedöffnung 124 mit einem zum Außengewinde korrespondierenden Innengewinde.

Durch Einschrauben des Lösezapfens 130 in die Lösegliedöffnung 124 wird der Kopplungseinsatz 56 in einer durch den Pfeil 140 definierten Richtung, die der Einführrichtung 80 entgegen gerichtet ist, aus der Kopplungseinsatzaufnahme 54 hinausgeschoben. Hierfür können unter Umständen große Kräfte erforderlich sein, da der Kopplungseinsatz 56 etwas im Bereich des rippenförmigen Verbindungsvorsprungs 116 komprimiert werden muss, so dass dieser aus der Verbindungselementausnehmung 114 heraus gelangen kann.

Um das Austreiben des Kopplungseinsatzes 56 aus der Kopplungseinsatzaufnahme 54 zu erleichtern, ist ausgehend von der seitlichen Kopplungseinsatzöffnung 98 an der Kopplungseinsatzaufnahme 54 bis kurz vor die Verbindungselementausnehmung 114 eine Vertiefung 142 vorgesehen, in die der Verbindungsvorsprung 116 eintauchen kann. So muss er nur eine kurze vorstehende Rippe 144 überwinden, und zwar sowohl beim Einschieben des Kopplungseinsatzes 56 in die Kopplungseinsatzaufnahme 54 als auch beim Herausdrücken des Kopplungseinsatzes 56 aus der Kopplungseinsatzaufnahme 54 heraus mit dem Löseinstrument 120.

Vorzugsweise ist der Extraktionsadapter 46 derart ausgebildet, dass nur die Kanten 86 und 88 des Kopplungseinsatzes 56 mit dem Kopplungskonus 38 in Kontakt treten können, nicht jedoch irgendwelche Kanten des Kopplungsgrundkörpers 52. So kann verhindert werden, dass der Kopplungskonus 38 durch den Kopplungsgrundkörper 52, welcher vorzugsweise ebenfalls aus einem Metall ausgebildet ist, beschädigt werden kann.

Das erste Kopplungselement 60 kann insbesondere identisch ausgebildet sein wie ein entsprechendes Kopplungselement an einem Raspelkörper, der mit dem Instrumentengriff 18 koppelbar ist, um die Kavität am Femurknochen zum Einsetzen des Prothesenschafts 14 vorzubereiten.

Das beschriebene Hüftgelenkendoprothesensystem ermöglicht eine schonende Extraktion eines Prothesenschafts 14 aus einem Femurknochen, und zwar ohne den Kopplungskonus 38 zu beschädigen.

### Bezugszeichenliste

- 10: Hüftgelenkendoprothesensystem
- 12: Instrument
- 14: Prothesenschaft
- 16: Hüftgelenkendoprothese
- 18: Instrumentengriff
- 20: Raspelgriff
- 22: Schlagkörper
- 24: Schlagfläche
- 26: Instrumentenkörper
- 28: Ende
- 30: Schaftkörper
- 32: Ende
- 34: Ende
- 36: Endfläche
- 38: Kopplungskonus
- 40: Endfläche
- 42: Ende
- 44: Halsabschnitt
- 46: Extraktionsadapter
- 48: Kopplungskörper
- 50: Kopplungsausnehmung
- 52: Kopplungsgrundkörper
- 54: Kopplungseinsatzaufnahme
- 56: Kopplungseinsatz
- 58: Endfläche
- 60: erstes Kopplungselement
- 62: Kopplungsvorsprung
- 64: Kopplungsvorsprunglängsachse
- 66: Ausnehmung
- 68: zweites Kopplungselement
- 70: Kopplungselementaufnahme
- 72: Sicherungsglied
- 74: Schwenkachse
- 76: Verriegelungsnase
- 78: seitliche Einführöffnung
- 80: Pfeil
- 82: Längsachse
- 84: untere Einführöffnung
- 86: Kante
- 88: Kante
- 90: Innenkontur
- 92: Innenkonus
- 94: Konuslängsachse
- 96: Querschnittsverengung
- 98: seitliche Kopplungseinsatzaufnahmeöffnung
- 100: untere Kopplungseinsatzaufnahmeöffnung
- 102: Hinterschneidung
- 104: Ringfläche
- 106: Verbindungseinrichtung
- 108: Rast- oder Schnappverbindungseinrichtung
- 110: erstes Verbindungselement
- 112: zweites Verbindungselement
- 114: Verbindungselementausnehmung
- 116: Verbindungsvorsprung
- 118: Öffnungsebene
- 120: Löseinstrument
- 122: Löseglied
- 124: Lösegliedöffnung
- 126: Länge
- 128: Dicke
- 130: Lösezapfen
- 132: Schaftlängsachse
- 134: Schaft
- 136: Endfläche
- 138: Griff
- 140: Pfeil
- 142: Vertiefung
- 144: Rippe

## Patentansprüche

1. Hüftgelenkendoprothesensystem (10) umfassend ein medizinisches Instrument (12) zum Extrahieren eines implantierten Prothesenschaftes (14) einer Hüftgelenkendoprothese (16) aus einem Femurknochen eines Patienten, welches medizinische Instrument (12) einen Kopplungskörper (48) mit einer Kopplungsausnehmung (50) zum Aufnehmen eines vom Prothesenschaft (14) abstehenden Kopplungskonus (38) aufweist, wobei der Kopplungskörper (48) einen Kopplungsgrundkörper (52) und einen in einer Kopplungseinsatzaufnahme (54) des Kopplungsgrundkörpers (52) aufgenommenen, die Kopplungsausnehmung (50) definierenden Kopplungseinsatz (56) umfasst, wobei das medizinische Instrument (12) eine Verbindungseinrichtung (106) umfasst zum kraft- und/oder formschlüssigen Verbinden des Kopplungseinsatzes (56) und des Kopplungsgrundkörpers (52) in einer Verbindungsstellung, **dadurch gekennzeichnet, dass** die Verbindungseinrichtung (106) in Form einer Rast- oder Schnappverbindungseinrichtung (108) ausgebildet ist.

2. Hüftgelenkendoprothesensystem nach Anspruch 1, **dadurch gekennzeichnet, dass** der Kopplungsgrundkörper (52) aus einem härteren Material ausgebildet ist als der Kopplungseinsatz (56).

3. Hüftgelenkendoprothesensystem nach einem der voranstehenden Ansprüche, **dadurch gekennzeichnet, dass**
a) der Kopplungsgrundkörper (52) aus einem Metall ausgebildet ist, insbesondere aus einem Instrumentenstahl
und/oder
b) der Kopplungseinsatz (56) aus einem Kunststoff ausgebildet ist, insbesondere aus Polyetheretherketon (PEEK) und/oder Polypropylen (PP) und/oder Polyphenylensulfon (PPSU),
und/oder
c) das medizinische Instrument (12) mindestens ein erstes Kopplungselement (60) umfasst zum Koppeln mit einem Instrumentengriff (18),
wobei insbesondere das erste Kopplungselement (60) in Form eines Kopplungsvorsprungs (62) ausgebildet ist.

4. Hüftgelenkendoprothesensystem nach einem der voranstehenden Ansprüche, **dadurch gekennzeichnet, dass** das medizinische Instrument (12) einen Instrumentengriff (18) umfasst, welcher insbesondere in Form eines Raspelgriffs (20) ausgebildet ist,
wobei insbesondere der Instrumentengriff (18) einen Schlagkörper (22) mit einer Schlagfläche (24) aufweist und dass der Schlagkörper (22) an einem proximalen Ende des Instrumentengriffs (18) angeordnet oder ausgebildet ist oder das proximale Ende des Instrumentengriffs (18) bildet.

5. Hüftgelenkendoprothesensystem nach Anspruch 4, **dadurch gekennzeichnet, dass** der Instrumentengriff (18) mindestens ein zweites Kopplungselement (68) umfasst zum kraft- und/oder formschlüssigen in Eingriff Bringen mit dem mindestens einen ersten Kopplungselement (60) in einer Koppelstellung,
wobei insbesondere
a) das mindestens eine zweite Kopplungselement (68) in Form einer Kopplungselementaufnahme (70) zum Aufnehmen des mindestens einen ersten Kopplungselements (60) ausgebildet ist
und/oder
b) das mindestens eine zweite Kopplungselement (68) an einem distalen Ende (28) des Instrumentengriffs (18) angeordnet oder ausgebildet ist oder das distale Ende (28) des Instrumentengriffs (18) bildet.

6. Hüftgelenkendoprothesensystem nach einem der voranstehenden Ansprüche, **dadurch gekennzeichnet, dass** die Kopplungsausnehmung (50) ausgebildet ist zum kraft- und/oder formschlüssigen Aufnehmen des Kopplungskonus (38).

7. Hüftgelenkendoprothesensystem nach einem der voranstehenden Ansprüche, **dadurch gekennzeichnet, dass** die Kopplungsausnehmung (50) eine seitliche Einführöffnung (78) aufweist zum Einschieben des Kopplungskonus (38) in die Kopplungsausnehmung (50) in einer Einführrichtung (80), welche quer, insbesondere senkrecht, zu einer durch den Kopplungskonus (38) definierten Längsachse (82) verläuft,
wobei insbesondere
a) die Kopplungsausnehmung (50) eine untere Einführöffnung (84) aufweist, welche in einer Richtung quer, insbesondere senkrecht, zur Einführrichtung (80) geöffnet ist,
wobei insbesondere der Kopplungsgrundkörper (52) den Kopplungseinsatz (56) derart umgibt, dass die seitliche Einführöffnung (78) und die untere Einführöffnung (84) frei zugänglich sind, wenn der Kopplungseinsatz (56) in die Kopplungseinsatzaufnahme (54) eingesetzt ist,
und/oder
b) der Kopplungseinsatz (56) die seitliche Einführöffnung (78) und/oder die untere Einführöffnung (84) begrenzende Kanten oder Übergangsbereiche definiert.

8. Hüftgelenkendoprothesensystem nach einem der voranstehenden Ansprüche, **dadurch gekennzeichnet, dass** die Kopplungsausnehmung (50) eine Innenkontur (90) aufweist, welche mindestens abschnittsweise die Form eines Innenkonus (92) aufweist,
wobei insbesondere
a) der Innenkonus (92) eine Konuslängsachse definiert, welche die untere Einführöffnung (84) durchsetzt
und/oder
b) ein Querschnitt des Innenkonus (92) in Richtung auf die untere Einführöffnung (84) hin zunimmt
und/oder
c) zwischen dem Innenkonus (92) und der unteren Einführöffnung (84) eine Querschnittsverengung (96) ausgebildet ist.

9. Hüftgelenkendoprothesensystem nach einem der voranstehenden Ansprüche, **dadurch gekennzeichnet, dass** die Kopplungseinsatzaufnahme (54) eine seitliche Kopplungseinsatzaufnahmeöffnung (98) und eine untere Kopplungseinsatzaufnahmeöffnung (100) aufweist.

10. Hüftgelenkendoprothesensystem nach Anspruch 9, **dadurch gekennzeichnet, dass**
a) die seitliche Kopplungseinsatzaufnahmeöffnung (98) ausgebildet ist zum Einschieben des Kopplungseinsatzes (56) in die Kopplungseinsatzaufnahme (54)
und/oder
b) die Kopplungseinsatzaufnahme (54) eine Hinterschneidung (102) aufweist, welche durch eine Verringerung eines freien Querschnitts der Kopplungseinsatzaufnahme (54) in Richtung auf die untere Kopplungseinsatzaufnahmeöffnung (100) hin gebildet ist
und/oder
c) das erste Kopplungselement (60) am Kopplungsgrundkörper (52) derart angeordnet ist, dass es entgegen oder im Wesentlichen entgegen der unteren Kopplungseinsatzaufnahmeöffnung (100) weist.

11. Hüftgelenkendoprothesensystem nach einem der voranstehenden Ansprüche, **dadurch gekennzeichnet, dass** der Kopplungseinsatz (56) in der Kopplungseinsatzaufnahme (54) kraft- und/oder formschlüssig gehalten ist.

12. Hüftgelenkendoprothesensystem nach einem der voranstehenden Ansprüche, **dadurch gekennzeichnet, dass** die Verbindungseinrichtung (106) erste und zweite Verbindungselemente (110, 112) umfasst, welche einerseits am Kopplungseinsatz (56) und andererseits am Kopplungsgrundkörper (52) ausgebildet sind, und dass die ersten und zweiten Verbindungselemente in der Verbindungsstellung kraft- und/oder formschlüssig in Eingriff stehen,
wobei insbesondere
a) das erste Verbindungselement (110) in Form einer Verbindungselementausnehmung (114) ausgebildet ist und dass das zweite Verbindungselement (112) in Form eines Verbindungsvorsprungs (116) ausgebildet ist
und/oder
b) sich das erste und/oder das zweite Verbindungselement (110, 112) parallel oder im Wesentlichen parallel zur seitlichen Kopplungseinsatzaufnahmeöffnung (98) und/oder zur seitlichen Einführöffnung (78) erstrecken
und/oder
c) das am Kopplungsgrundkörper (52) in der Kopplungseinsatzaufnahme (54) ausgebildete Verbindungselement (110) in Richtung auf die untere Kopplungseinsatzaufnahmeöffnung (110) hin weisend angeordnet oder ausgebildet ist.

13. Hüftgelenkendoprothesensystem nach einem der voranstehenden Ansprüche, **gekennzeichnet durch** ein Löseinstrument (120) zum Lösen des Kopplungseinsatzes (56) aus der Kopplungseinsatzaufnahme (54) des Kopplungsgrundkörpers (52),
wobei insbesondere das Löseinstrument (120) ein Löseglied (122) umfasst, dass der Kopplungsgrundkörper (52) eine Lösegliedöffnung (124) umfasst und dass das Löseglied (122) eine Länge (126) aufweist, welche größer ist als eine Dicke (128) des Kopplungsgrundkörpers (52) im Bereich der Lösegliedöffnung (124).

14. Hüftgelenkendoprothesensystem nach einem der Ansprüche 1 bis 12, **dadurch gekennzeichnet, dass** das Hüftgelenkendoprothesensystem ein Löseinstrument (120) zum Lösen des Kopplungseinsatzes (56) aus der Kopplungseinsatzaufnahme (54) des Kopplungsgrundkörpers (52) umfasst, dass das Löseinstrument (120) ein Löseglied (122) umfasst, dass der Kopplungsgrundkörper (52) eine Lösegliedöffnung (124) umfasst und dass das Löseglied (122) eine Länge (126) aufweist, welche größer ist als eine Dicke (128) des Kopplungsgrundkörpers (52) im Bereich der Lösegliedöffnung (124) und dass
a) die Lösegliedöffnung (124) der seitlichen Kopplungseinsatzaufnahmeöffnung (98) gegenüberliegend angeordnet oder ausgebildet ist und/oder
b) das Löseglied (122) in Form eines vom Löseinstrument abstehenden Lösezapfens (130) ausgebildet ist
und/oder
c) das Löseglied (122) mit einem Außengewinde versehen ist und dass die Lösegliedöffnung (124) mit einem zum Außengewinde korrespondierenden Innengewinde versehen ist.

15. Hüftgelenkendoprothesensystem nach einem der voranstehenden Ansprüche, **gekennzeichnet durch** einen einen abstehenden Kopplungskonus (38) aufweisenden Prothesenschaft (14) einer Hüftgelenkendoprothese (16).

## Claims

1. Hip joint endoprosthesis system (10), comprising a medical instrument (12) for extracting an implanted prosthesis stem (14) of a hip joint endoprosthesis (16) from a femur bone of a patient, which medical instrument (12) has a coupling body (48) with a coupling recess (50) for accommodating a coupling cone (38) protruding from the prosthesis stem (14), wherein the coupling body (48) comprises a coupling base body (52) and a coupling insert (56) which is accommodated in a coupling insert receptacle (54) of the coupling base body (52) and defines the coupling recess (50), wherein the medical instrument (12) comprises a connecting device (106) for connecting the coupling insert (56) and the coupling base body (52) in a connecting position in a force- and/or positive-locking manner, **characterized in that** the connecting device (106) is configured in the form of a latching or snapping connecting device (108).

2. Hip joint endoprosthesis system in accordance with claim 1, **characterized in that** the coupling base body (52) is formed of a harder material than the coupling insert (56).

3. Hip joint endoprosthesis system in accordance with any one of the preceding claims, **characterized in that**
a) the coupling base body (52) is formed of a metal, in particular of an instrument steel
and/or
b) the coupling insert (56) is formed of a plastic, in particular of polyetheretherketone (PEEK) and/or polypropylene (PP) and/or polyphenylene sulfone (PPSU)
and/or
c) the medical instrument (12) comprises at least one first coupling element (60) for coupling to an instrument handle (18),
wherein in particular the first coupling element (60) is configured in the form of a coupling projection (62).

4. Hip joint endoprosthesis system in accordance with any one of the preceding claims, **characterized in that** the medical instrument (12) comprises an instrument handle (18), which in particular is configured in the form of a rasp handle (20),
wherein in particular the instrument handle (18) comprises a striking body (22) with a striking face (24), and **in that** the striking body (22) is arranged or formed on a proximal end of the instrument handle (18) or forms the proximal end of the instrument handle (18).

5. Hip joint endoprosthesis system in accordance with claim 4, **characterized in that** the instrument handle (18) comprises at least one second coupling element (68) for bringing into engagement in a force- and/or positive-locking manner with the at least one first coupling element (60) in a coupling position,
wherein in particular
a) the at least one second coupling element (68) is configured in the form of a coupling element receptacle (70) for accommodating the at least one first coupling element (60)
and/or
b) the at least one second coupling element (68) is arranged or formed on a distal end (28) of the instrument handle (18) or forms the distal end (28) of the instrument handle (18).

6. Hip joint endoprosthesis system in accordance with any one of the preceding claims, **characterized in that** the coupling recess (50) is configured to accommodate the coupling cone (38) in a force- and/or positive-locking manner.

7. Hip joint endoprosthesis system in accordance with any one of the preceding claims, **characterized in that** the coupling recess (50) has a lateral insertion opening (78) for inserting the coupling cone (38) into the coupling recess (50) in an insertion direction (80) which runs transversely, in particular perpendicularly, to a longitudinal axis (82) defined by the coupling cone (38),
wherein in particular
a) the coupling recess (50) has a lower insertion opening (84) which is open in a direction transverse, in particular perpendicular, to the insertion opening (80),
wherein in particular the coupling base body (52) surrounds the coupling insert (56) in such a way that the lateral insertion opening (78) and the lower insertion opening (84) are freely accessible when the coupling insert (56) is inserted into the coupling insert receptacle (54)
and/or
b) the coupling insert (56) defines edges or transition regions delimiting the lateral insertion opening (78) and/or the lower insertion opening (84).

8. Hip joint endoprosthesis system in accordance with any one of the preceding claims, **characterized in that** the coupling recess (50) has an inner contour (90) which has the form of an inner cone (92) at least in sections,
wherein in particular
a) the inner cone (92) defines a longitudinal cone axis which passes through the lower insertion opening (84)
and/or
b) a cross section of the inner cone (92) increases in the direction toward the lower insertion opening (84)
and/or
c) a cross-sectional narrowing (96) is formed between the inner cone (92) and the lower insertion opening (84).

9. Hip joint endoprosthesis system in accordance with any one of the preceding claims, **characterized in that** the coupling insert receptacle (54) has a lateral coupling insert receptacle opening (98) and a lower coupling insert receptacle opening (100).

10. Hip joint endoprosthesis system in accordance with claim 9, **characterized in that**
a) the lateral coupling insert receptacle opening (98) is configured for the insertion of the coupling insert (56) into the coupling insert receptacle (54)
and/or
b) the coupling insert receptacle (54) has an undercut (102) which is formed by a reduction of a free cross section of the coupling insert receptacle (54) in the direction toward the lower coupling insert receptacle opening (100)
and/or
c) the first coupling element (60) is arranged on the coupling base body (52) in such a way that it points counter to or substantially counter to the lower coupling insert receptacle opening (100).

11. Hip joint endoprosthesis system in accordance with any one of the preceding claims, **characterized in that** the coupling insert (56) is held in the coupling insert receptacle (54) in a force- and/or positive-locking manner.

12. Hip joint endoprosthesis system in accordance with any one of the preceding claims, **characterized in that** the connecting device (106) comprises first and second connecting elements (110, 112) which are formed on the coupling insert (56) on the one hand and on the coupling base body (52) on the other hand, and **in that** the first and second connecting elements are in engagement in a force- and/or positive-locking manner in the connecting position,
wherein in particular
a) the first connecting element (110) is configured in the form of a connecting element recess (114), and **in that** the second connecting element (112) is configured in the form of a connecting projection (116)
and/or
b) the first and/or the second connecting element (110, 112) extend in parallel or substantially in parallel to the lateral coupling insert receptacle opening (98) and/or to the lateral insertion opening (78) and/or
c) the connecting element (110) formed on the coupling base body (52) in the coupling insert receptacle (54) is arranged or formed pointing in the direction toward the lower coupling insert receptacle opening (110).

13. Hip joint endoprosthesis system in accordance with any one of the preceding claims, **characterized in that** the hip joint endoprosthesis system comprises a release instrument (120) for releasing the coupling insert (56) from the coupling insert receptacle (54) of the coupling base body (52),
wherein in particular the release instrument (120) comprises a release member (122), **in that** the coupling base body (52) comprises a release member opening (124), and **in that** the release member (122) has a length (126) which is greater than a thickness (128) of the coupling base body (52) in the region of the release member opening (124).

14. Hip joint endoprosthesis system in accordance with any one of claims 1 to 12, **characterized by** a release instrument (120) for releasing the coupling insert (56) from the coupling insert receptacle (54) of the coupling base body (52), in that the release instrument (120) comprises a release member (122), in that the coupling base body (52) comprises a release member opening (124), and in that the release member (122) has a length (126) which is greater than a thickness (128) of the coupling base body (52) in the region of the release member opening (124), and in that
a) the release member opening (124) is arranged or formed opposite the lateral coupling insert receptacle opening (98)
and/or
b) the release member (122) is configured in the form of a release pin (130) projecting from the release instrument
and/or
c) the release member (122) is provided with an external thread, and in that the release member opening (124) is provided with an internal thread corresponding to the external thread.

15. Hip joint endoprosthesis system in accordance with any one of the preceding claims, **characterized by** a prosthesis stem (14) of a hip joint endoprosthesis (16), which prosthesis stem (14) has a projecting coupling cone (38).

## Revendications

1. Système d'endoprothèse d'articulation de la hanche (10) comprenant un instrument médical (12) pour extraire une tige de prothèse implantée (14) d'une endoprothèse d'articulation de la hanche (16) d'un os fémur d'un patient, lequel instrument médical (12) présente un corps de couplage (48) avec un évidement de couplage (50) pour loger un cône de couplage (38) faisant saillie de la tige de prothèse (14), où le corps de couplage (48) comprend un corps de base de couplage (52) et un insert de couplage (56) définissant l'évidement de couplage (50), logé dans un logement d'insert de couplage (54) du corps de base de couplage (52), où l'instrument médical (12) comprend un dispositif de liaison (106) pour la liaison par assemblage de force et/ou de forme de l'insert de couplage (56) et du corps de base de couplage (52) dans une position de liaison, **caractérisé en ce que** le dispositif de liaison (106) est réalisé sous forme d'un dispositif de liaison par verrouillage ou par encliquetage (108).

2. Système d'endoprothèse d'articulation de la hanche selon la revendication 1, **caractérisé en ce que** le corps de base de couplage (52) est réalisé en un matériau plus dur que l'insert de couplage (56).

3. Système d'endoprothèse d'articulation de la hanche selon l'une des revendications précédentes, **caractérisé en ce que**
a) le corps de base de couplage (52) est réalisé en un métal, en particulier en un acier d'instrument
et/ou
b) l'insert de couplage (56) est réalisé en une matière plastique, en particulier en polyétheréthercétone (PEEK) et/ou polypropylène (PP) et/ou polyphénylènesulfone (PPSU),
et/ou
c) l'instrument médical (12) comprend au moins un premier élément de couplage (60) pour le couplage à un manche d'instrument (18),
où en particulier le premier élément de couplage (60) est réalisé sous forme d'une saillie de couplage (62).

4. Système d'endoprothèse d'articulation de la hanche selon l'une des revendications précédentes, **caractérisé en ce que** l'instrument médical (12) comprend un manche d'instrument (18) qui est réalisé en particulier sous forme d'un manche de râpe (20),
où en particulier le manche d'instrument (18) présente un corps de frappe (22) avec une surface de frappe (24) et **en ce que** le corps de frappe (22) est disposé ou réalisé à une extrémité proximale du manche d'instrument (18) ou forme l'extrémité proximale du manche d'instrument (18).

5. Système d'endoprothèse d'articulation de la hanche selon la revendication 4, **caractérisé en ce que** le manche d'instrument (18) comprend au moins un second élément de couplage (68) pour mettre en prise par assemblage de force et/ou de forme avec le au moins un premier élément de couplage (60) dans une position de couplage,
où en particulier
a) le au moins un second élément de couplage (68) est réalisé sous forme d'un logement d'élément de couplage (70) pour loger le au moins un premier élément de couplage (60) et/ou
b) le au moins un second élément de couplage (68) est disposé ou réalisé à une extrémité distale (28) du manche d'instrument (18) ou forme l'extrémité distale (28) du manche d'instrument (18).

6. Système d'endoprothèse d'articulation de la hanche selon l'une des revendications précédentes, **caractérisé en ce que** l'évidement de couplage (50) est réalisé pour loger le cône de couplage (38) par assemblage de force et/ou de forme.

7. Système d'endoprothèse d'articulation de la hanche selon l'une des revendications précédentes, **caractérisé en ce que** l'évidement de couplage (50) présente une ouverture d'insertion latérale (78) pour insérer le cône de couplage (38) dans l'évidement de couplage (50) dans une direction d'insertion (80), qui s'étend transversalement, en particulier perpendiculairement, à un axe longitudinal (82) défini par le cône de couplage (38),
où en particulier
a) l'évidement de couplage (50) présente une ouverture d'insertion inférieure (84) qui est ouverte dans une direction transversale, en particulier perpendiculaire, à la direction d'insertion (80),
où en particulier le corps de base de couplage (52) entoure l'insert de couplage (56) de telle sorte que l'ouverture d'insertion latérale (78) et l'ouverture d'insertion inférieure (84) sont librement accessibles lorsque l'insert de couplage (56) est inséré dans le logement d'insert de couplage (54), et/ou
b) l'insert de couplage (56) définit les bords limitant ou les domaines de transition de l'ouverture d'insertion latérale (78) et/ou de l'ouverture d'insertion inférieure (84).

8. Système d'endoprothèse d'articulation de la hanche selon l'une des revendications précédentes, **caractérisé en ce que** l'évidement de couplage (50) présente un contour interne (90) qui, au moins en sections, présente la forme d'un cône interne (92),
où en particulier
a) le cône interne (92) définit un axe longitudinal de cône, qui traverse l'ouverture d'insertion inférieure (84)
et/ou
b) une section transversale du cône interne (92) augmente en direction de l'ouverture d'insertion inférieure (84)
et/ou
c) un rétrécissement de section transversale (96) est réalisé entre le cône interne (92) et l'ouverture d'insertion inférieure (84).

9. Système d'endoprothèse d'articulation de la hanche selon l'une des revendications précédentes, **caractérisé en ce que** le logement d'insert de couplage (54) présente une ouverture de logement d'insert de couplage latérale (98) et une ouverture de logement d'insert de couplage inférieure (100).

10. Système d'endoprothèse d'articulation de la hanche selon la revendication 9, **caractérisé en ce que**
a) l'ouverture de logement d'insert de couplage latérale (98) est réalisée pour l'insertion de l'insert de couplage (56) dans le logement d'insert de couplage (54)
et/ou
b) le logement d'insert de couplage (54) présente une contre-dépouille (102) qui est formée par une réduction d'une section transversale libre du logement d'insert de couplage (54) en direction de l'ouverture de logement d'insert de couplage inférieure (100)
et/ou
c) le premier élément de couplage (60) est disposé sur le corps de base de couplage (52) de telle manière qu'il est tourné de manière opposée ou sensiblement opposée à l'ouverture de logement d'insert de couplage inférieure (100).

11. Système d'endoprothèse d'articulation de la hanche selon l'une des revendications précédentes, **caractérisé en ce que** l'insert de couplage (56) est maintenu par assemblage de force et/ou de forme dans le logement d'insert de couplage (54).

12. Système d'endoprothèse d'articulation de la hanche selon l'une des revendications précédentes, **caractérisé en ce que** le dispositif de liaison (106) comprend des premier et second éléments de liaison (110, 112), qui sont réalisés d'une part sur l'insert de couplage (56) et d'autre part sur le corps de base de couplage (52), et **en ce que** les premier et second éléments de liaison sont en prise par assemblage de force et/ou de force dans la position de liaison,
où en particulier
a) le premier élément de liaison (110) est réalisé sous forme d'un évidement d'élément de liaison (114) et le second élément de liaison (112) est réalisé sous forme d'une saillie de liaison (116)
et/ou
b) le premier et/ou le second élément de liaison (110, 112) s'étendent parallèlement ou sensiblement parallèlement à l'ouverture de logement d'insert de couplage latérale (98) et/ou à l'ouverture d'insertion latérale (78)
et/ou
c) l'élément de liaison (110) réalisé sur le corps de base de couplage (52) dans le logement d'insert de couplage (54) est disposé ou réalisé en étant tourné en direction de l'ouverture de logement d'insert de couplage inférieure (110).

13. Système d'endoprothèse d'articulation de la hanche selon l'une des revendications précédentes, **caractérisé par** un instrument de libération (120) pour libérer l'insert de couplage (56) du logement d'insert de couplage (54) du corps de base de couplage (52),
où en particulier l'instrument de libération (120) comprend un organe de libération (122), en ce que le corps de base de couplage (52) comprend une ouverture d'organe de libération (124) et en ce que l'organe de libération (122) présente une longueur (126) qui est supérieure à une épaisseur (128) du corps de base de couplage (52) dans le domaine de l'ouverture d'organe de libération (124).

14. Système d'endoprothèse d'articulation de la hanche selon l'une des revendications 1 à 12, **caractérisé en ce que** le système d'endoprothèse d'articulation de la hanche comprend un instrument de libération (120) pour libérer l'insert de couplage (56) du logement d'insert de couplage (54) du corps de base de couplage (52), **en ce que** l'instrument de libération (120) comprend un organe de libération (122), **en ce que** le corps de base de couplage (52) comprend une ouverture d'organe de libération (124) et **en ce que** l'organe de libération (122) présente une longueur (126) qui est supérieure à une épaisseur (128) du corps de base de couplage (52) dans le domaine de l'ouverture d'organe de libération (124) et **en ce que**
a) l'ouverture d'organe de libération (124) est disposée ou réalisée en face de l'ouverture de logement d'insert de couplage latérale (98)
et/ou
b) l'organe de libération (122) est réalisé sous forme d'une cheville de libération (130) faisant saillie de l'instrument de libération
et/ou
c) l'organe de libération (122) est muni d'un filetage externe et **en ce que** l'ouverture d'organe de libération (124) est munie d'un filetage interne correspondant au filetage externe.

15. Système d'endoprothèse d'articulation de la hanche selon l'une des revendications précédentes, **caractérisé par** une tige de prothèse (14) d'une endoprothèse d'articulation de la hanche (16) présentant un cône de couplage en saillie (38).
